Europäisches Patentamt

European Patent Office

Office européen des brevets

(11) Veröffentlichungsnummer: **0 125 556**
**B1**

(12) **EUROPÄISCHE PATENTSCHRIFT**

(45) Veröffentlichungstag der Patentschrift: **28.09.88**

(51) Int. Cl.⁴: **G 01 N 21/89**, G 01 N 33/36

(21) Anmeldenummer: **84104834.1**

(22) Anmeldetag: **30.04.84** -

(54) **Verfahren und Vorrichtung zur Kontrolle der Farbaufnahmefähigkeit von Textilen.**

(30) Priorität: **04.05.83 DE 3316172**

(43) Veröffentlichungstag der Anmeldung:
**21.11.84 Patentblatt 84/47**

(45) Bekanntmachung des Hinweises auf die
Patenterteilung:
**28.09.88 Patentblatt 88/39**

(84) Benannte Vertragsstaaten:
**CH FR GB IT LI**

(56) Entgegenhaltungen:
**CH-A- 431 135**
**CH-A- 622 349**
**DE-A-1 938 083**
**DE-A-3 129 808**
**DE-B-2 426 866**
**DE-C-2 655 973**

**Die Akte enthält technische Angaben, die nach
dem Eingang der Anmeldung eingereicht
wurden und die nicht in dieser Patentschrift
enthalten sind.**

(73) Patentinhaber: **Mahlo GmbH & Co. KG
Donaustrasse 12
D-8424 Saal/Donau (DE)**

(72) Der Erfinder hat auf seine Nennung verzichtet

(74) Vertreter: **Reinländer, Claus, Dr. et al
MEISSNER, BOLTE & PARTNER
Widenmayerstrasse 48 Postfach 86 06 24
D-8000 München 86 (DE)**

Anmerkung: Innerhalb von neun Monaten nach der Bekanntmachung des Hinweises auf die Erteilung des europäischen
Patents im Europäischen Patentblatt kann jedermann beim Europäischen Patentamt gegen das erteilte europäische
Patent Einspruch einlegen. Der Einspruch ist schriftlich einzureichen und zu begründen. Er gilt erst als eingelegt,
wenn die Einspruchsgebühr entrichtet worden ist (Art. 99(1) Europäisches Patentübereinkommen).

Courier Press, Leamington Spa, England.

**Beschreibung**

Ein großes und für den Qualitätsausfall entscheidendes Problem beim Färben von Textilien ist der ungleichmäßige Farbausfall über Breite und Länge der zu färbenden Stücke. Farbunterschiede, sogenannte Farbabläufe, zwischen der Mitte der Bahn und den Kantenzonen sind nicht selten; desgleichen treten solche Unterschiede auch häufig zwischen Anfang und Ende einer Textilbahn auf. Bei der Konfektionierung derartiger gefärbter Artikel äußern sich dann solche Farbabweichungen in nicht akzeptierbaren Farbunterschieden, z.B. innerhalb eines Kleidungsstückes.

Die Färbefehler dieser Art können verschiedene Ursachen haben. Je nach angewandten Färbeverfahren sind maschinentechnische Fehler, Bedienungsfehler, insbesondere aber auch Ungleichmäßigkeiten in der zu färbenden Rohware möglich. Ein Grund für Farbabläufe ist die Aufbringung unterschiedlich großer Farbflottenmengen auf die Ware, bedingt etwa durch nicht gleichmäßige Abquetschung der Ware selbst. Es sind deshalb Einrichtungen bekannt, die die Kontrolle der über die Breite der Ware verteilten Wassermenge zu kontrollieren erlauben, z.B. mit Hilfe von Feuchtigkeitsmeßgeräten nach dem Mikrowellen-Absorptionsverfahren (DE-PS 26 55 973). Zur Überwachung der Farbgleichmäßigkeit wurden auch farbmetrische Meßgeräte entwickelt, die bereits an der laufenden Warenbahn, aber natürlich nach der Trocknung der Farbe, die Farben objektiv zu messen gestatten.

Die Messung der Verteilung der wässrigen Farbflotte und die mögliche Regelung dieser Verteilung berücksichtigt jedoch nur eine der vielfältigen Ursachen für die Farbungleichmäßigkeit und wie die Erfahrungen der Färber lehren nicht einmal die entscheidende. Dagegen bringt die Rohware selbst sehr häufig Eigenschaften mit, die eine unterschiedliche Farbflottenaufnahme verursachen. Solche sind beispielsweise ein ungleichmäßiges Flächengewicht, vor allem aber auch nicht gleichmäßige Farbaufnahmeeigenschaften, hervorgerufen beispielsweise durch Schwankungen in der Struktur der Textilien, Faserrauhigkeit, der Größe und der Verteilung der Kapillaren zwischen den Fasern, des dadurch bedingten hygroskopischen Verhaltens und dgl.

Es ist deshalb für den Färber sehr wichtig, die Farbaufnahmeeigenschaften eines angelieferten ungefärbten Stückes bereits vor der Färbung zu kennen, damit besonders ungleichmäßige Partien von vornherein z.B. für eine Uni-Färbung gar nicht erst vorgesehen werden. Solche Stücke können dagegen zum Druck oder zu anderer Verwendung disponiert werden, bei der Farbabläufe keine gravierende Rolle spielen.

In vielen Fällen gehen Färber so vor, daß sie den Mittenbereich und die Randbereiche der zu färbenden Ware nacheinander an den Mund halten und durchblasen bzw. saugen, um dadurch die Luftdurchlässigkeit des Gewebes zu prüfen. Je nach dem gefühlsmäßigen Prüfungsergebnis wird dann die Abquetschwalze in der Mitte bzw. am Rand mehr oder weniger festpressend eingestellt. Diese Prüfmethode ist naturgemäß ungenau und bei einer laufenden Ware nicht anwendbar.

Der Erfindung liegt die Aufgabe zugrunde, ein Verfahren und eine Vorrichtung zur Durchführung des Verfahrens zu schaffen, um die Farbaufnahmefähigkeit einer laufenden Bahn einer textilen Ware zu kontrollieren.

Diese Aufgabe wird verfahrensmäßig dadurch gelöst, daß man die Durchlässigkeit der textilen Bahn über ihre Breite gegenüber elektromagnetischen Wellen, vorzugsweise Lichtwellen, mißt, mit einem vorgegebenen Standard vergleicht und den Vergleichswert zur Kontrolle der Farbaufnahmefähigkeit heranzieht.

Das Verfahren läßt sich durch eine Vorrichtung durchführen, die mehrere Paare von jeweils einem Sender an der einen Seite und mindestens einem gegenüberliegenden Empfänger an der anderen Seite der Bahn aufweist, wobei die Sende-/Empfänger-Paare quer zur Bahn bewegbar sind und im wesentlichen nebeneinander, quer zur Laufrichtung der Bahn angeordnet sind.

Die Prüfung einer textilen Bahn mittels elektromagnetischer Wellen ist an sich aus der CH-A-431 135 oder für die Prüfung von Folien aus der DE-B-24 26 866 bekannt. In diesen Fällen handelt es sich aber um die sogenannte "Warenschau", bei der die textile Ware auf (sichtbare) strukturelle Fehler, wie Löcher, fehlende Kettfäden, fehlende Maschen, fehlende Schußfäden, Ölflecken, eingebundene Fremdkörper, Schlingen etc. überwacht werden soll. Demzufolge wird von den hierfür verwendeten Geräten eine möglichst gute (optische) Auflösung gefordert. Aufgrund dieser guten optischen Auflösung der verwendeten Geräte ist es nicht möglich, diese nun zur Lösung einer ganz anderen Aufgabe, nämlich zur Prüfung der Farbaufnahmefähigkeit einer Ware einzusetzen, da das generierbare Signal auf alle möglichen Fehler-Strukturparameter empfindlich reagiert und der für die Kontrolle der Farbaufnahmefähigkeit entscheidende Meßwert in diesen Signalen untergeht. Nachdem man bislang die Farbaufnahmefähigkeit über die mechanische Messung der Luftdurchlässigkeit (Prüfung durch den Färber selbst) gemessen hat und diesem mechanischen parameter die allergrößte Bedeutung beimaß, ist es besonders überraschend, daß sich die Farbaufnahmefähigkeit auch über elektromagnetische Wellen prüfen läßt.

Im folgenden wird anhand eines Beispieles beschrieben, wie eine derartige Meßanordnung ausgeführt werden kann.

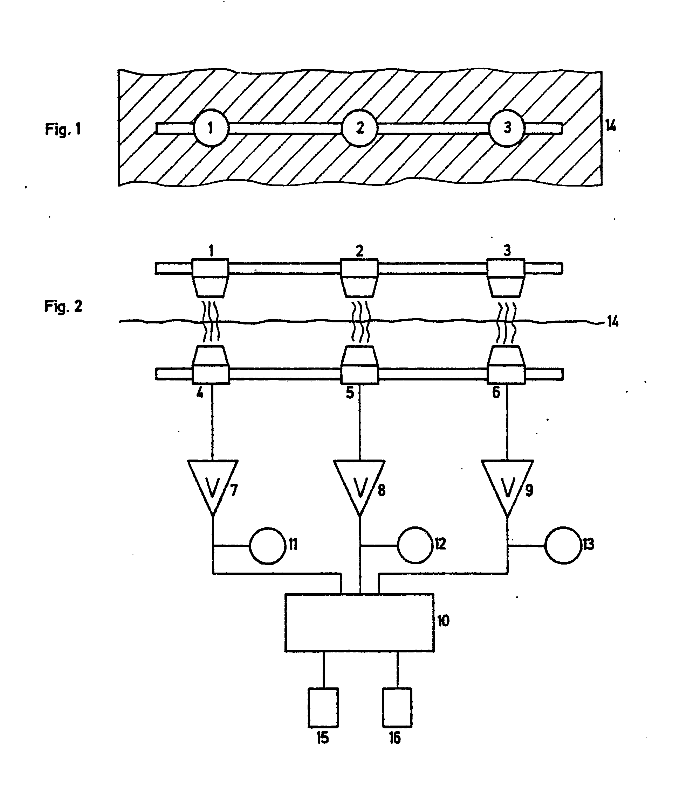

In Fig. 1 ist in Draufsicht und in Fig. 2 im Schnitt ein Schema der Meßanordnung zur Messung von Farbaufnahmeeigenschaften der Ware dargestellt.

In den Sendern 1 bis 3 wird Licht erzeugt und auf die textile Warenbahn 14 aufgestrahlt. Die Empfänger 4 bis 6 können Lichtmengenmeßgeräte sein. Sie wandeln die durch das Textil hindurchtretenden Energien in elektrische Signale um. Die empfangenen Energien werden mit den jeweils zugehörigen Sendeenergien verglichen. Daraus wird die Dämpfung bestimmt, die ein Maß für die Durchlässigkeit des Prüfguts ist. Außerdem werden in einer Schaltung 10 die Dämpfungen der drei Strecken (1-4, 2-5 und 3-6) miteinander verglichen, um Ungleichmäßigkeiten in der Durchlässigkeit festzustellen. Mit den Anzeigegeräten 11, 12 und 13 wird schließlich die Durchlässigkeit des Prüfguts an drei Stellen (Mitte und beide Kanten) angezeigt. Sie kann gleichzeitig ausgedruckt (15) oder geschrieben (16) werden; die Drucker-oder Schreiber-Protokolle werden zweckmäßigerweise mit den textilen Stücken als Unterlagen für die Färberei weitergegeben. 7, 8 und 9 sind Verstärker.

Da zur Erzielung des erfindungsgemäßen Effektes nur relative Meßwerte, also der Vergleich gegenüber einem vorgebenen Standard über die Bahnlänge interessieren, werden auch nur genügend gleichartige Meßanordnungen benötigt, nicht aber reproduzierbare absolute Meßwerte. Die Auswahl des anzuwendenden Meßmediums richtet sich nach den Gegebenheiten und Eigenschaften des textilen Prüfguts, die allerdings erheblich unterschiedlich sein können. So werden sehr offene Textilien mit Licht als Medium geprüft.

Wegen der geforderten Gleichheit der Eigenschaften der einzelnen Meßstrecken empfiehlt sich u.U. die Verwendung nur eines Meßaggregats, das aber quer zur Bahn bewegt wird und dabei ein lückenloses Profil der Durchlässigkeitsverhältnisse liefert. Auch zwei oder mehr symmetrisch zur Mitte der Bahn bewegte Meßaggregate sind denkbar, deren Signale ständig paarweise miteinander verglichen werden.

## Patentansprüche

1. Verfahren zur Kontrolle der Farbaufnahmefähigkeit einer laufenden Bahn einer textilen Ware, insbesondere offenen Ware, wobei man die Durchlässigkeit der textilen Bahn über ihre Breite gegenüber elektromagnetischen Wellen, vorzugsweise Lichtwellen, mißt, mit einem vorgegebenen Standard vergleicht und den Vergleichswert zur Kontrolle der Farbaufnahmefähigkeit heranzieht.

2. Vorrichtung zur Durchführung des Verfahrens nach Anspruch 1, mit mehreren Paaren von jeweils einem Sender an der einen Seite und einem gegenüberliegenden Empfänger an der anderen Seite der Bahn, wobei die Sender(1, 2, 3) und Empfänger (4, 5, 6) Paare quer zur Bahn (14) bewegbar und im wesentlichen nebeneinander, quer zur Laufrichtung der Bahn angeordnet sind.

3. Vorrichtung nach Anspruch 2, dadurch gekennzeichnet, daß die Sender-/Empfänger-Paare (1-4, 2-5, 3-6) symmetrisch zur Mitte der Bahn hin- und herbewegbar sind.

## Revendications

1. Procédé pour le contrôle de la capacité d'absorption de colorant d'une bande d'un article textile en voie de défiler, en particulier une étoffe lâche, dans lequel on mesure la perméabilité de la bande textile, sur sa largeur, à des ondes électromagnétiques, de préférence des ondes lumineuses, la compare à une valeure de référence préétablie et utilise la valeur comparative pour le contrôle de la capacité d'absorption de colorant.

2. Dispositif pour la mise en oeuvre du procédé selon la revendication 1, équipé de plusieurs paires formées chacune d'un émetteur disposé d'un côté et d'un récepteur lui faisant face de l'autre côté de la bande, les paires d'émetteurs (1, 2, 3) et de récepteurs (4, 5, 6) étant déplaçables transversalement à la bande (14) et disposées sensiblement côte à côte, transversalement à la direction de défilement de la bande.

3. Dispositif selon la revendication 2, caractérisé en ce que les paires d'émetteur-récepteur (1-4, 2-5, 3-6) sont déplaçables d'un mouvement alternatif symétriquement par rapport au milieu de la bande.

## Claims

1. Process for controlling the dye receptivity of a moving web of textile material in particular loosely woven material, whereby one measures the permeability in respect of electromagnetic waves, preferably light waves, of the textile web across its width, compares it with a preselected standard and uses the result of the comparison for controlling the dye receptivity.

2. Device for implementing the process according to claim 1, with several pairs comprising one transmitter on one side and an opposite receiver on the other side of the web each, whereby the pairs of transmitters (1, 2, 3) and receivers (4, 5, 6) can be displaced at right angles to web (14) and are substantially arranged next to one another, at right angles to the direction in which the web moves.

3. Device according to claim 2, characterised in that the pairs of transmitters and receivers (1-4, 2-5, 3-6) can be moved to and fro symmetrically in respect of the middle of the web.

**Fig. 1**

**Fig. 2**